Europäisches Patentamt

European Patent Office

Office européen des brevets

(19)

(11) Numéro de publication : **0 308 278 B1**

# FASCICULE DE BREVET EUROPEEN

(12)

(45) Date de publication du fascicule du brevet :
08.01.92 Bulletin 92/02

(51) Int. Cl.$^5$ : **A61K 7/48, A61K 7/06**

(21) Numéro de dépôt : 88401902.7

(22) Date de dépôt : 22.07.88

(54) Utilisation cosmétique de dérivés N-acétyles ou N-propionyles de la proline, de l'hydroxyproline et/ou du mélange d'acides aminés résultant de l'hydrolyse du collagène.

(30) Priorité : 02.09.87 FR 8712183

(43) Date de publication de la demande :
22.03.89 Bulletin 89/12

(45) Mention de la délivrance du brevet :
08.01.92 Bulletin 92/02

(84) Etats contractants désignés :
AT BE CH DE ES FR GB GR IT LI LU NL SE

(56) Documents cités :
EP-A- 0 197 506
EP-A- 0 197 510
FR-A- 1 520 356
FR-A- 2 237 616
FR-A- 2 503 153
INTERNATIONAL JOURNAL OF COSMETIC
SCIENCE, vol. 8, 1986, pages 73-80; M.C. MAR-
TINI et al.: "Influence du butyrylhydroxyproline sur le développement des fibroblastes en
culture"

(73) Titulaire : LES ETABLISSEMENTS GIVAUDAN
LAVIROTTE & CIE
56 rue Paul Cazeneuve
F-69008 Lyon (FR)

(72) Inventeur : Berger, Christian
La Forestière No. 3
F-69130 Ecully (FR)
Inventeur : Gacon, Paul
21, rue Barthélémy Thimonier
F-69160 Tassin la Demi Lune (FR)

(74) Mandataire : Cazes, Jean-Marie et al
RHONE-POULENC INTERSERVICES Service
Brevets Chimie 25, quai Paul Doumer
F-92408 Courbevoie Cédex (FR)

EP 0 308 278 B1

Jouve, 18, rue Saint-Denis, 75001 PARIS

## Description

La présente invention concerne l'application à titre de produits cosmétiques des dérivés N-acétylés ou N-propionylés de la proline, de l'hydroxyproline et/ou du mélange d'acides aminés obtenu par hydrolyse du collagène, des sels de ces dérivés, ainsi que les compositions les renfermant.

Des dérivés N-acylés de certains acides aminés ont déjà été décrits pour leurs applications cosmétiques. Ainsi le brevet français 2503153 enseigne l'emploi, notamment dans le domaine cosmétique, de dérivés d'acides aminés obtenus par combinaison de la chaîne butyrique avec les fonctions acylables de la proline, de l'hydroxyproline et de l'hydroxylysine. Lesdits dérivés sont doués d'un pouvoir de pénétration cutanée dû à leur caractère hydrosoluble et à la nature de la structure lipidoprotidique. Leur rôle cosmétique réside en une acidification de la peau. Il est en effet connu que la stabilité physiologique de la peau est en grande partie liée à la balance acido-basique de la couche superficielle du stratum-corneum. C'est pourquoi, dans de nombreuses compositions cosmétiques, on retrouve de tels produits acidifiants.

Cependant la demanderesse a pu constater que de tels dérivés butyrylés présentent une forte odeur, ce qui les rend peu aptes à toute application cosmétique. En effet, l'un des critères les plus importants que doit posséder un produit d'usage cosmétique est son absence d'odeur désagréable.

Le brevet FR 1520356 décrit un procédé de traitement des protéines biologiques impliquant un bloquage de leurs fonctions amines et ayant pour effet notamment, de les rendre inodores. Toutefois ce document ne mentionne aucune application des composés ainsi obtenus.

En outre, certains de ces dérivés butyrylés, décrits dans le brevet français 2503153, sont peu stables : ils noircissent au cours du temps.

Afin d'obvier ces inconvénients, la demanderesse a trouvé l'application en cosmétique de dérivés d'acides aminés qui ne présentent pas d'odeur désagréable, qui sont stables au cours du temps, et qui bien que ne possédant pas de chaîne grasse, présentent un pouvoir de pénétration tissulaire.

En outre, et d'une façon tout à fait surprenante, la demanderesse a pu constater que lesdits dérivés présentent en plus des avantages précités, un pouvoir oxygénant et régénérant des cellules cutanées remarquable.

Or, il est connu que l'activité métabolique d'une substance, et par là, son potentiel cosmétique, peut se traduire au niveau cutané par une augmentation de la consommation d'oxygène, ce qui contribue à la régénérescence cellulaire.

De plus, lesdits dérivés permettent de diminuer de façon significative l'attaque enzymatique de l'élastine par l'élastase. De ce fait, ces dérivés peuvent être considérés comme actifs dans le domaine de l'élasticité cutanée.

Un des buts de la présente invention est donc l'application à titre de produits cosmétiques, de composés ne présentant pas d'odeur désagréable, stables au cours du temps, pénétrant dans la peau, qui en outre sont pourvus d'un remarquable pouvoir oxygénant et régénérant des cellules cutanées et permettent à la peau de conserver son élasticité.

Pour ce faire, la présente invention consiste en l'application à titre de produits cosmétiques des dérivés N-acétylés ou N-propionylés de la proline, de l'hydroxyproline et/ou du mélange d'acides aminés obtenu par hydrolyse du collagène, ainsi que les sels métalliques ou de bases organiques de ces dérivés.

D'autres caractéristiques et avantages de l'invention apparaîtront plus clairement à la lecture de la description qui va suivre.

Les dérivés N-acétylés et N-propionylés de la proline et de l'hydroxyproline peuvent être préparés, de façon connue en soi, par acylation en milieu aqueux ou organique desdits acides aminés, à l'aide de dérivés activés de l'acide acétique et de l'acide propionique. De tels dérivés activés sont par exemple, les anhydrides acétique ou propionique ou les chlorures d'acétyle ou de propionyle. Généralement l'opération s'effectue à une température comprise entre 0 et 100°C.

Les dérivés N-acétylés et N-propionylés obtenus peuvent être éventuellement purifiés par des méthodes telles que la cristallisation ou la chromatographie.

Dans le cadre de la présente invention, on entend par collagène, les protéines d'origine animale diverse contenues dans la substance fondamentale des tissus de soutien.

L'hydrolyse du collagène peut être réalisée, de manière connue, par chauffage à des températures comprises entre 60 et 130°C dudit collagène placé dans un milieu aqueux acide. L'acidification dudit milieu peut être obtenu par des acides forts tels que l'acide sulfurique ou l'acide chlorhydrique.

Le mélange d'acides aminés obtenu peut être ensuite neutralisé au moyen d'une base forte telle que la soude ou la potasse. Dans une étape ultérieure, ledit mélange d'acides aminés en solution aqueuse peut alors être acylé selon le même procédé que celui utilisé pour l'acylation de la proline et de l'hydroxyproline, à l'aide donc des dérivés activés des acides acétique et propionique précités.

Le mélange d'acides aminés N-acétylés ou N-propionylés peut être purifié par exemple par distillation et entraînement à l'eau, sous vide, des éventuelles impuretés. Cette opération de purification peut se faire à des températures comprises entre 30 et 60°C et de préférence comprises entre 40 et 50°C.

L'invention s'étend également aux sels des dérivés N-acétylés ou N-propionylés de la proline, de l'hydroxyproline ou des mélanges d'acides aminés obtenus par hydrolyse du collagène. Ces sels peuvent être préparés par réaction desdits dérivés N-acétylés ou N-propionylés avec des bases organiques ou inorganiques, ou avec des dérivés métalliques.

A titre de bases organiques ou inorganiques pouvant entrer dans le cadre de la présente invention, on peut citer la potasse, la soude, la chaux, la magnésie, l'ammoniaque ; la triéthanolamine, la diéthylamine, le morphine, la lysine, l'histidine, l'arginine, l'ornithine, la choline.

A titre de sels obtenus par réaction avec des dérivés métalliques, on peut citer les sels de zinc, d'aluminium, de cuivre, de cobalt, de fer et de manganèse.

Dans le cadre de la présente invention, les dérivés N-acétylés ou N-propionylés de la proline, de l'hydroxyproline et/ou du mélange d'acides aminés obtenu par hydrolyse du collagène, ainsi que les sels desdits dérivés, peuvent à titre de produits cosmétiques, entrer dans la formulation de compositions cosmétiques. A titre de compositions cosmétiques, on peut citer les crèmes, les laits, les mousses, les aérosols, les gels, les sticks, les huiles, les émulsions, les savons ainsi que les lotions aqueuses ou hydroalcooliques.

Dans lesdites compositions cosmétiques, les dérivés N-acétylés et N-propionylés précités ou les sels de ces dérivés sont présents dans des proportions de l'ordre de 0,1 à 20% et de préférence de 0,5 à 10% en poids par rapport au poids total de la composition.

Les compositions cosmétiques entrant dans le cadre de la présente invention sont stables, exemptes d'odeurs désagréables et de plus confèrent aux cellules cutanées un remarquable pouvoir oxygénant et régénérant, montrant leur fort potentiel cosmétique et permettent à la peau de conserver son élasticité.

Les exemples suivants sont donnés à titre indicatif.

Exemples 1 à 4 :

Ces exemples sont relatifs à des compositions cosmétiques selon l'invention. Les principes actifs figurant dans ces compositions sont la N-propionyl L hydroxy-4 proline, la N-propionyl L proline ou le dérivé N-propionylé du mélange d'acides aminés obtenu par hydrolyse du collagène.

Exemple 1

## Crème régénératrice

Composition :

| | | |
|---|---|---|
| | Tefose 1500 • | 10 g |
| | Depelargonate de propylène glycol | 5 g |
| A | Huile de vaseline fluide | 5 g |
| | Alcool cétylique | 3 g |
| | Parahydroxybenzoate de méthyle | 0,15 g |
| | Parahydroxybenzoate de méthyle sodé | 0,15 g |
| B | Principe actif | 10 g |
| | Carbonate acide de sodium | QS pour pH 5 |
| | Eau | QS pour 100 g |
| | Parfum | 0,1 g |

<u>Procédé de préparation de la crème</u>

On chauffe A et B séparément à 70°C.
Puis on mélange B dans A sous agitation modérée. On refroidit à 30°C et on ajoute le parfum.
Cette crème régénératrice est destinée aux soins du visage et confère un effet antirides.

<u>Exemple 2</u>

<u>Crème de massage</u>

<u>Composition</u> :

| | | | |
|---|---|---|---|
| | | Cetasal● | 15 g |
| | | Labrafil 2130 CS● | 5 g |
| | | Vaseline blanche | 3 g |
| A | | Huile de silicone | 1 g |
| | | Parleam | 2 g |
| | | Parahydroxybenzoate de méthyle | 0,1 g |
| | | Sorbitol | 2 g |
| | | Parahydroxybenzoate de méthyle sodé | 0,1 g |
| B | | Principe actif | 10 g |
| | | Carbonate acide de sodium | QS pour pH 5 |
| | | Eau | QS pour 100 g |
| | | Parfum | 0,1 g |

<u>Procédé de préparation de la crème :</u>

On chauffe A et B séparément à 70°C, puis on mélange B dans A. On refroidit à 30°C et on ajoute le parfum.
Cette crème de massage est utilisable pour la prévention de l'apparition des vergetures ou pour l'amélioration de leur aspect.

Exemple 3

## Lait corporel

Composition :

| A | Huile de vaseline fluide | 10 g |
| | Palmitate d'isopropyle | 4 g |
| | Huile de tournesol | 5 g |
| | Alcool cétylique | 1 g |
| | Tween 60 | 2,6 g |
| | Span 60 | 2,4 g |
| | Kathon CG (solution) ✷ | 0,1 g |
| | Principe actif | 5 g |
| B | Carbonate acide de sodium | QS pour pH 5 |
| | Eau | QS pour 100 g |
| | | |
| | Parfum | 0,1 g |

Procédé de préparation du lait :

On chauffe A et B séparément à 70°C, puis on mélange B dans A sous agitation rapide. On refroidit à 30°C et on ajoute le parfum.

Ce lait d'application quotidienne est destiné à entretenir et améliorer l'élasticité cutanée.

Exemple 4

## Masque gel

Composition :

| Extrait glycolique de camomille | 7 g |
| Glycérol | 10 g |
| Carbopol 940 | 1 g |
| Triéthanolamine | QS pour pH 6 |
| Principe actif | 5 g |
| Kathon CG ✷ (solution) | 0,1 g |
| Colorant | QS |
| Parfum hydrosoluble | 0,3 g |
| Eau | QS pour 100 g |

Procédé de préparation du masque gel :

On disperse le Carbopol dans les 3/4 du volume total d'eau. On met en solution dans le reste d'eau tous les composants de la formule à l'exception de la triéthanolamine.

On ajoute ensuite lentement ce mélange au Carbopol dispersé et on neutralise par la triéthanolamine.

Ce masque gel peut être appliqué en couche épaisse et maintenu en contact avec la peau pendant 20 minutes. Après rinçage, on sèche rapidement, puis on applique une crème émolliente.

5

Exemples 5 à 9

Ces exemples permettent de mettre en évidence le pouvoir oxygénant et régénérant des cellules cutanées par action des dérivés N-acétylés et N-propionylés selon l'invention.

Les tests utilisés dans ces exemples sont basés sur la technique de mesure des pressions d'oxygène ou "technique d'oxygraphie". Ils ont pu être réalisés sur des cellules hépatiques de souris car on sait en effet que des mesures de consommation d'oxygène faites sur de telles cellules sont tout à fait révélatrices des consommations d'oxygène des cellules cutanées et donc de leur faculté à se régénérer.

Ces tests ont été réalisés de la manière suivante : la mesure de la consommation d'oxygène est effectuée à l'aide d'une électrode à oxygène BECKMANN 39550, qui est une électrode en verre, de type Clarke, permettant de suivre les variations de concentration en oxygène dissous. On adapte sur l'électrode une cellule de mesure constituée d'un tube de verre cylindrique ; l'électrode ainsi adaptée plonge dans un bain-marie thermostaté entre 30 et 50°C ;

On introduit dans la cellule 3,6 ml de liquide de Ringer contenant un broyat de foie de souris. La concentration en broyat de foie de souris est de 30 mg/ml de liquide de Ringer. On effectue alors une mesure témoin de consommation d'oxygène sur 10 minutes. Puis on ajoute dans la cellule un volume du produit à tester, dilué dans du liquide de Ringer.

Une nouvelle mesure de consommation d'oxygène est réalisée sur 10 minutes.

L'introduction du produit selon l'invention à tester, produit une variation de la consommation d'oxygène.

Cette variation est obtenue par la formule suivante :

$$\text{var.} = \frac{B - A}{A} \times 100$$

dans cette formule

— var. est le pourcentage de variation de la consommation d'oxygène.

A est la valeur en ppm de la consommation d'oxygène au moment où l'on introduit le produit à tester.

B est la valeur en ppm de la consommation d'oxygène au bout de 10 minutes suivant l'introduction du produit à tester.

Les résultats obtenus dans les exemples 5 et 6 figurent dans le tableau I suivant :

## Tableau I

| ex | produit testé | concentration du produit testé dans la cellule (% en volume par rapport au volume total contenu dans la cellule) | variation de la consommation d'oxygène |
|---|---|---|---|
| 5 | C$_3$Co | 1 % | 220 % |
| 6 | C$_3$Pro | 1 % | 78 % |
| 7 | C$_3$HP | 1 % | 110 % |
| 8 | C$_3$HP | 1,43 % | 135 % |
| 9 | C$_2$HP | 1 % | 42 % |

Dans ce tableau : C$_3$Co est le dérivé N-propionylé du mélange d'acides aminés obtenu par hydrolyse du collagène
C$_3$Pro est la N-propionyl L proline
C$_3$HP est la N-propionyl L hydroxy-4 proline
C$_2$HP est la N-acétyl L hydroxy-4 proline.

Ces exemples montrent clairement que l'introduction des composés selon l'invention, favorisent l'oxygénation cellulaire.

Exemple 10 :

Les essais réalisés dans cet exemple ont permis de mettre en évidence le pouvoir protecteur de l'élastine vis-à-vis de l'élastase, conféré par les composés selon l'invention.

Les essais sont basés sur le principe de la diffusion de l'élastate dans un gel d'agarose à 1% et dans lequel est dispersée de l'élastine insoluble colorée par l'orcéine.

Après 48 heures d'incubation dans une étuve à 37°C, on mesure la surface des zones claires correspondant à la zone de digestion enzymatique.

Les essais ont été ici réalisés sur un gel témoin (essai A) et sur des gels comportant des composés selon l'invention, à savoir :

• essai B : N-propionyl proline (C$_3$ Pro)
• essai C : N-propionyl L hydroxy-4 proline (C$_3$HP)
• essai D : dérivé N-propionylé du mélange d'acides aminés obtenu par hydrolyse du collagène (C$_3$Co)

Les gels sont obtenus de la manière suivante :

7

EP 0 308 278 B1

—gel de l'essai A :

0,25 g de poudre d'Agarose Prolabor sont complétés à 25 ml par du tampon TRIS à pH 8,3 ; (le TRIS pH 8,3 est préparé à partir d'un mélange TRIS-CaCl$_2$, dont le pH est ajusté à la valeur désirée, au moyen d'HCl 0,1 N).

Le mélange est placé au bain-marie bouillant pendant 15 à 20 minutes jusqu'à obtention d'une solution limpide. On maintient cette solution au bain-marie à 40°C puis on la mélange avec 0,0375 g d'élastine-orcéine Sigma E-1500.

On répartit 20 g de ce mélange dans deux boites de Petri de 90 mm de diamètre.

—gels des essais B, C et D :

0,25 g d'agarose sont mélangés à 18 ml de tampon TRIS pH 10,4, ce mélange étant complété à 25 ml par de l'eau. On place le mélange au bain-marie bouillant, puis à 40°C, comme précédemment.

On mélange ensuite à 40°C, 25 g du gel ainsi préparé, 0,0375 g d'élastine-orcéine et, soit 0,125 g de C$_3$ Pro (essai B) ou de C$_3$HP (essai C), soit 0,25 g de C$_3$Co (essai D).

Le pH du gel est alors de 8,3.

On répartit ensuite 20 g de chaque mélange dans deux boites de Petri de 90 mm de diamètre.

Depôt de l'élastase :

Dans chacun des gels obtenus de la manière décrite ci-dessus, on creuse à l'emporte-pièce un puits de 3 mm de diamètre. On ajoute dans chaque puits 5 l d'élastase pancréatique porcine E-1250 (activité 73 U/mg protéine).

Puis on place les boites de Pétri à 37°C pendant 48 heures. On mesure alors la surface de la zone de digestion autour de chaque puits.

Cette zone est une tache blanche sur fond bleu.

Les résultats des essais figurent dans le tableau II suivant :

## Tableau II

| essai | produit testé | surface en mm$^2$ | q % |
|-------|---------------|-------------------|-----|
| A | - | 300 | - |
| B | C$_3$ Pro | 213 | - 29 % |
| C | C$_3$ HP | 226 | - 24,66 % |
| D | C$_3$ Co | 201 | - 33 % |

Dans ce tableau q % représente la diminution de l'attaque de l'élastine par l'élastase.

Cette valeur est obtenue par la formule suivante :

$$q \% = (\frac{S1}{S2} - 1) \times 100$$

où S1 est la surface d'une zone de digestion dans un gel comportant un composé selon l'invention et S2 est la surface d'une zone de digestion dans le gel témoin.

8

## Revendications

1. Application à titre de produit cosmétique pour la peau des dérivés N-acétylés ou N-propionylés de la proline, de l'hydroxyproline et/ou du mélange d'acides aminés obtenu par hydrolyse du collagène, ainsi que les sels métalliques ou de bases organiques de ces dérivés, lesdits dérivés étant actifs contre l'élastase et préservant l'élasticité cutanée.

2. Compositions cosmétiques pour la peau caractérisées en ce qu'elles renferment des dérivés N-acétylés ou N-propionylés de la proline, de l'hydroxyproline et/ou du mélange d'acides aminés obtenu par hydrolyse du collagène, ou des sels métalliques ou de bases organiques de ces dérivés, lesdits dérivés étant actifs contre l'élastase et préservant l'élasticité cutanée.

3. Compositions cosmétiques pour la peau selon la revendication 2 caractérisées en ce qu'elles renferment de 0,1 à 20% en poids par rapport au poids total de la composition, desdits dérivés N-acétylés ou N-propionylés ou de leurs sels.

4. Compositions cosmétiques pour la peau selon la revendication 3 caractérisées en ce qu'elles renferment de 0,5 à 10% en poids par rapport au poids total de la composition desdits dérivés N-acétylés ou N-propionylés ou de leurs sels.

5. Compositions cosmétiques pour la peau selon l'une des revendications 2 à 4 caractérisées en ce qu'elles se présentent sous forme de crèmes, de laits, de mousses, d'aérosols, de gels, de sticks, d'huiles, d'émulsions, de savons, de lotions aqueuses ou hydroalcooliques.

## Patentansprüche

1. Anwendung von N-Acetyl- oder N-Propionyl-Derivaten von Prolin, Hydroxyprolin und/oder einem Gemisch von Aminosäuren, das erhalten worden ist durch Hydrolyse von Collagen, sowie den Metallsalzen oder Salzen von organischen Basen dieser Derivate, wobei diese Derivate aktiv sind gegen Elastase und die Elastizität der Haut erhalten als Hautkosmetikum.

2. Kosmetische Mittel für die Haut, gekennzeichnet dadurch, daß sie umfassen N-Acetyl- oder N-Propionyl-Derivate von Prolin, Hydroxyprolin und/oder Gemischen von Aminosäuren, erhalten durch Hydrolyse von Collagen, oder die Metallsalze oder Salze mit organischen Basen dieser Derivate, wobei diese Derivate aktiv sind gegen Elastase und die Elastizität der Haut erhalten.

3. Kosmetische Mittel für die Haut nach Anspruch 2, dadurch gekennzeichnet, daß sie 0,1 bis 20 Gew.-%, bezogen auf das Gesamtwicht des Mittels, dieser N-Acetyl- oder N-Propionyl-Derivate oder deren Salzen enthalten.

4. Kosmetische Mittel nach Anspruch 3, dadurch gekennzeichnet, daß sie 0,5 bis 10 Gew.-%, bezogen auf das Gesamtgewicht des Mittels, dieser N-Acetyl- oder N-Propionyl-Derivate oder deren Salzen enthalten.

5. Kosmetische Mittel nach einem der Ansprüche 2 bis 4, dadurch gekennzeichnet, daß sie in Form von Cremes, als Milch, in Form von Schäumen, Aerosolen, Gels, Stiften, Ölen, Emulsionen, Seifen, wäßrigen oder wäßrig alkoholischen Lotionen vorliegen.

## Claims

1. Application as a cosmetic product for the skin of N-acetyl or N-propionyl derivatives of proline, of hydroxyproline and/or of the mixture of amino acids obtained by hydrolysis of collagen, as well as metal salts or salts of organic bases of these derivatives, the said derivatives being active against elastase and preserving the elasticity of the skin.

2. Cosmetic compositions for the skin, characterised in that they contain N-acetyl or N-propionyl derivatives of proline, of hydroxyproline and/or of the mixture of amino acids obtained by hydrolysis of collagen, or metal salts or salts of organic bases of these derivatives, the said derivatives being active against elastase and preserving the elasticity of the skin.

3. Cosmetic compositions for the skin according to Claim 2, characterised in that they contain from 0.1 to 20% by weight, relative to the total weight of the composition, of the said N-acetyl or N-propionyl derivatives or their salts.

4. Cosmetic compositions for the skin according to Claim 3, characterised in that they contain from 0.5 to 10% by weight, relative to the total weight of the composition, of the said N-acetyl or N-propionyl derivatives or their salts.

5. Cosmetic compositions for the skin according to one of Claims 2 to 4, characterised in that they take the form of creams, milks, foams, aerosols, gels, sticks, oils, emulsions, soaps or aqueous or aqueous-alcoholic lotions.